(19) Europäisches Patentamt / European Patent Office / Office européen des brevets

(11) **EP 2 027 817 B1**

(12) **FASCICULE DE BREVET EUROPEEN**

(45) Date de publication et mention
de la délivrance du brevet:
**24.02.2016 Bulletin 2016/08**

(51) Int Cl.:
*A63B 21/06* (2006.01)          *A63B 24/00* (2006.01)
*A63B 71/06* (2006.01)          *A61B 5/11* (2006.01)
*A61B 5/22* (2006.01)           *A63B 23/04* (2006.01)

(21) Numéro de dépôt: **07114873.8**

(22) Date de dépôt: **23.08.2007**

(54) **Accéléromètre et procédé de commande adapté**

Beschleunigungsmesser und angepasste Steuerung

Accelerometer and adapted control method

(84) Etats contractants désignés:
**AT BE BG CH CY CZ DE DK EE ES FI FR GB GR
HU IE IS IT LI LT LU LV MC MT NL PL PT RO SE
SI SK TR**

(43) Date de publication de la demande:
**25.02.2009 Bulletin 2009/09**

(73) Titulaire: **Myotest SA
1950 Sion (CH)**

(72) Inventeurs:
• **Flaction, Patrick
1965 Savièse (CH)**
• **Bezinge, Alex
1965 Savièse (CH)**
• **Quièvre, Jacques
77540 Rozay en Brie (FR)**

(74) Mandataire: **P&TS SA (AG, Ltd.)
Av. J.-J. Rousseau 4
P.O. Box 2848
2001 Neuchâtel (CH)**

(56) Documents cités:
**EP-A- 1 834 583          WO-A-2005/055815
WO-A-2007/036611          GB-A- 2 422 790
JP-A- 2006 320 533          US-A1- 2006 191 335**

**Description**

Domaine technique

La présente invention concerne un accéléromètre utilisé pour la mesure de paramètres physiologiques musculaires à partir de tests courts.

Etat de la technique

Il existe de nombreux documents décrivant des accéléromètres pour la mesure de performances sportives. La plupart des accéléromètres existants sont destinés à des exercices de longue durée, par exemple pour évaluer la distance parcourue ou la consommation calorique pendant une séance de jogging ou une randonnée cycliste. D'autres accéléromètres liés par exemple à un gyroscope sont associés à un accessoire dont on souhaite déterminer la trajectoire, par exemple pour exercer un swing de golf ou un revers au tennis. Il existe par ailleurs des accéléromètres employés pour mesurer un impact dans un sport de combat. Il existe aussi des dispositifs assez similaires pour détecter les chutes de personnes âgées, le temps qu'elles passent assis, debout ou couchées, etc.

Aucun de ces dispositifs ne permet d'obtenir des paramètres physiologiques musculaires de base comme la force, la vitesse ou la puissance d'un groupe musculaire.

US5788655 (Omron) décrit un appareil destiné à être fixé sur le corps et muni d'un accéléromètre et d'un afficheur LCD. L'appareil mesure en permanence les déplacements du porteur pour déterminer son niveau d'activité physique et des d'autres grandeurs dépendant du métabolisme, par exemple la consommation de calorie journalière de l'utilisateur. Ce type d'appareil est utile pour mesurer de manière plus objective le niveau de sédentarité de patients. Il est cependant inadapté à l'entraînement musculaire et à la mesure d'efforts brefs, et ne permet pas de déterminer par exemple la puissance maximale d'un groupe musculaire de l'athlète.

WO2005074795 (Nokia) décrit un terminal de mesure muni d'un accéléromètre et attaché sur le corps d'un athlète. Les données de mesure sont évaluées pour fournir une grandeur représentative de l'intensité de l'effort fourni. A nouveau, le but est de déterminer le niveau d'activité sur une longue période, par exemple une journée ou une semaine.

WO03/032826 (Philips) décrit un système comparable et muni d'un accéléromètre à trois axes pour déterminer le niveau d'activité physique d'un patient. Le dispositif proposé affiche des grandeurs telles que le taux métabolique quotidien, la dépense énergétique quotidienne ou la dépense d'énergie induite par l'exercice. Cet appareil permet donc de mesurer des accélérations sur une période de plusieurs heures, voire de plusieurs jours.

US5474083 décrit un système destiné à surveiller les mouvements de levé de charge par un patient. Le système emploie des électrodes pour mesurer l'activité des muscles du patient au cours du mouvement, ainsi qu'un détecteur de mouvement de la charge. Une alarme est déclenchée en cas de mouvement inapproprié. Ce système est utile pour empêcher les accidents provoqués par le lever de charges incorrect, ou pour exercer des personnes à soulever des charges sans se blesser. Il est cependant inapproprié à la mesure de la performance musculaire du sportif. Par ailleurs, l'usage d'électrodes rend son emploi peu pratique.

US6148280 (Virtual Technologies) décrit un dispositif muni d'accéléromètres et de gyroscopes disposés sur tout le corps d'un athlète. Les données fournies par plusieurs capteurs sont transmises à un ordinateur personnel qui permet d'analyser la trajectoire et d'autres caractéristiques du mouvement. Ce système est complexe, car il met en oeuvre plusieurs capteurs, y compris des gonomiètres coûteux, relativement fragiles. La connexion des capteurs entre eux et vers l'ordinateur externe renchérit le dispositif, et rend son installation malaisée. Il est adapté à l'entraînement de mouvements précis, par exemple un swing de golf, mais ne permet pas de déterminer directement la capacité musculaire développée par le sportif au cours de ce mouvement.

DE446302 décrit un accéléromètre utilisé dans les sports de combat pour la mesure de l'accélération de la surface de frappe. L'appareil n'est pas portable et convient uniquement aux sports de combat tels que box, karaté, etc. Un ordinateur externe doit être employé pour évaluer les résultats de mesure et les afficher. Il n'est pas programmable et ne peut être utilisé que pour un seul type d'exercice. En outre le document US2006/0191335 qui représente l'état de la technique le plus proche divulgue la préambule de la revendication 1. Finalement le document EP1834583 qui fait partie de l'état de la technique au sens de l'article 54(3) CBE divulgue également la préambule de la revendication 1.

Bref résumé de l'invention

Un but de la présente invention est de proposer un accéléromètre amélioré qui permet de mesurer très facilement, avec des tests simples et courts, des valeurs physiologiques musculaires telles que la force, la puissance, la vitesse, etc.

Par test court, on entend dans ce texte un test durant typiquement quelques minutes, par exemple un test comportant un ou plusieurs sauts ou levers de poids, la durée totale du test durant de préférence au maximum quelques minutes

Un autre but de l'invention est de proposer un accéléromètre polyvalent, adapté à différents tests et au calcul de différentes grandeurs, ainsi qu'un procédé de commande adapté à cet accéléromètre.

Selon l'invention, ces buts sont atteints notamment au moyen d'un procédé pour obtenir les valeurs physiologiques musculaires d'un utilisateur à l'aide d'un accéléromètre programmable, le procédé comportant les opérations suivantes :

- sélection par l'utilisateur du type de test à effectuer;
- mesures d'une séquence de données d'accélération au cours du test ;
- émission d'un signal à la fin du test, la fin du test étant déterminée en vérifiant une condition dépendant du type de test sélectionné ;
- affichage d'une valeur calculée à partir desdites mesures successives d'accélération et dépendant du type de test sélectionné.

Ce procédé présente notamment l'avantage d'utiliser un seul accéléromètre programmable pour déterminer différentes grandeurs à l'aide de tests très divers. Par ailleurs, la fin d'un test est déterminée automatiquement, à l'aide de critères qui dépendent du test sélectionné. On évite ainsi les contraintes des dispositifs qui obligent l'utilisateur à effectuer une manipulation pour signaler à l'accéléromètre que l'acquisition de donnée doit être interrompue et que l'on souhaite afficher des grandeurs mesurées ou calculées.

Brève description des figures

Des exemples de mise en oeuvre de l'invention sont indiqués dans la description illustrée par les figures annexées dans lesquelles :

La figure 1 illustre un accéléromètre pour l'évaluation de capacité musculaire à l'aide de tests courts.

La figure 2 est un schéma-bloc des principaux composants électroniques de l'accéléromètre de la figure 1.

La figure 3 illustre la force, la vitesse et la puissance que peut déployer un athlète en fonction de la charge déplacée.

La figure 4 est un diagramme de flux des étapes initiales du programme exécuté par l'accéléromètre de l'invention.

La figure 5 est un diagramme de flux du programme exécuté par l'accéléromètre de l'invention selon le test sélectionné.

Exemple(s) de mode de réalisation de l'invention

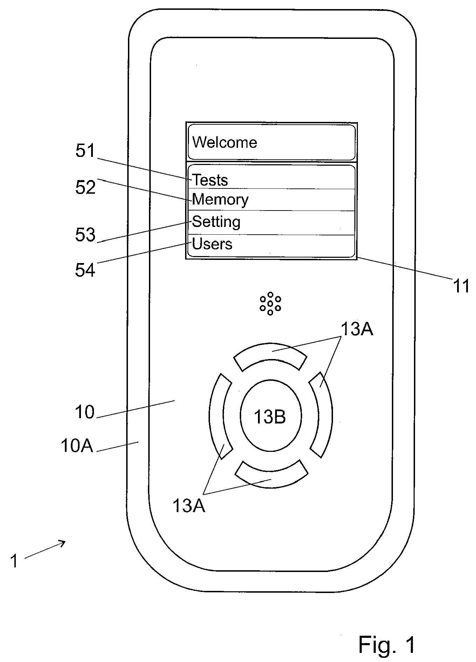

Un exemple d'accéléromètre 1 selon l'invention est illustré sur la figure 1. Le dispositif 1 comporte un boîtier 10, par exemple un boîtier en plastique ou en aluminium eloxé de moins de 100 grammes (y compris le contenu) que l'athlète peut fixer, selon le test effectué, sur son corps ou sur la charge soulevée à l'aide de moyens de fixation, par exemple d'une bande velcro, d'une sangle, etc. ou en le glissant dans une poche ou un étui. Le boîtier est de préférence étanche et permet une utilisation en plein air et en résistant à la sueur de l'athlète. Une protection souple 10A tout autour du boîtier permet de le protéger des chocs.

Le dispositif comporte en outre un affichage 11, par exemple un écran à cristaux liquides matriciel, qui permet d'afficher des positions de menu 51 à 54, l'état de la mémoire 55, l'état de la batterie 56, ainsi que des indications alphanumériques avant, pendant ou après le test. Des organes de commande 13 permettent de naviguer dans les menus affichés, de sélectionner des options, d'introduire des données, et de sélectionner les résultats affichés. Dans l'exemple préférentiel illustré, les organes de commande comprenant quatre boutons de navigation 13A (haut -bas - gauche - droite) et un bouton 13B pour confirmer les choix effectués.

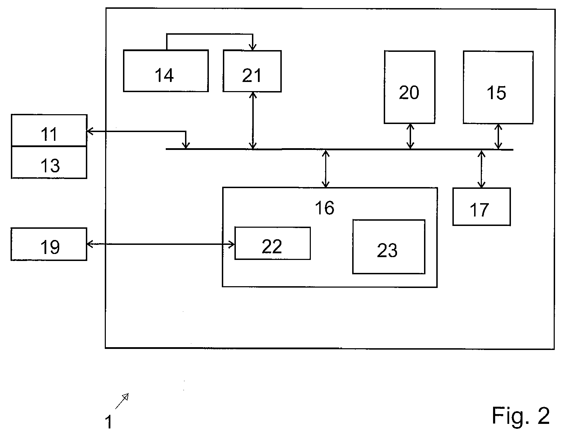

La figure 2 est un schéma-bloc illustrant les principaux composants électroniques du dispositif 1. Outre les organes externes déjà décrits en relation avec la figure 10, il comporte un capteur d'accélération, de préférence un capteur tri-axial 14. Le capteur est réalisé sous forme de composant MEMS et lié à un convertisseur analogique-numérique 21, ou intégrant directement un tel convertisseur, pour fournir des séquences de mesures d'accélération selon trois axes. Le capteur 14 peut comporter un ou plusieurs axes privilégiés, offrant une précision ou une résolution de mesure supérieure aux autres axes. Cet axe privilégié sera par exemple approximativement vertical lorsque le dispositif se trouve dans sa position d'utilisation habituelle, afin d'améliorer la précision de mesure selon la direction verticale. L'emploi d'un accéléromètre à trois axes permet cependant de s'affranchir de la nécessité d'un ajustement précis de l'axe de mesure avec la direction d'accélération privilégiée.

De préférence, l'accéléromètre est en outre calibré lors de sa production afin de vérifier les offsets et autres erreurs systématiques selon chaque axe, et d'introduire des facteurs correctifs correspondants dans l'accéléromètre et/ou dans un logiciel traitant les données de l'accéléromètre.

De préférence, le dispositif est dépourvu de gyroscope afin de réduire son coût, sa consommation et le volume de données produites; l'usage d'un gyroscope à un axe, ou même à trois axes, pourrait cependant être envisagé pour certains types de tests de capacité musculaire, par exemple afin de suivre un déplacement dans un espace tridimensionnel et comportant des rotations.

Le dispositif 1 est de préférence autonome électriquement et alimenté par exemple à l'aide d'une batterie 15 de préférence rechargeable, par exemple au travers de la prise USB 19 ou en la sortant du boîtier. La batterie 15 alimente notamment un microprocesseur 16 ou un microcontrôleur muni d'une mémoire 23 RAM et/ou EEPROM. Le microprocesseur exécute un programme (firmware) de préférence chargé en EEPROM, et remplaçable au travers de l'interface 19 ou via un lecteur de support de données, pour analyser les données de mesure fournies par l'accéléromètre 14 et commander l'affichage 11 afin d'afficher les grandeurs désirées.

Le dispositif 1 comporte encore une horloge en temps réel (RTC) 20 qui permet notamment de mesurer des intervalles de temps $\Delta t$. La période d'échantillonnage et les intervalles de temps doivent être suffisamment courts pour mesurer de manière dynamique des sauts. De manière générale, des intervalles de 5 millisecondes sont suffisants pour des tests de charge relativement lents, tandis que des intervalles de 1 à 2 millisecondes au maximum sont préférables pour des tests de saut qui ont une dynamique beaucoup plus importante.

Le dispositif 1 comporte aussi un transducteur sonore 17 (buzzer 17 ou haut-parleur) pour générer des signaux de commande, d'erreur ou d'autres sons. Le transducteur 17 permet de communiquer des instructions à l'athlète même lorsqu'il ne regarde pas l'écran ; les signaux émis peuvent être des signaux courts (bips, bip-bip, bîîîîp, etc.), des signaux polyphoniques ou des commandes vocales pré-enregistrées ou générées par un synthétiseur vocal. Les signaux sonores peuvent aussi être transmis par une interface sans fil vers un casque ou un lecteur de musique.

Un module d'entrée-sortie (UART) 22 permet au microprocesseur 16 d'échanger des données avec des dispositifs externes, par exemple pour reprogrammer le firmware ou pour transmettre des résultats de mesure ou des paramètres d'utilisateur à un ordinateur personnel, un téléphone mobile ou un autre dispositif externe de traitement de données. Le module 22 permet également d'introduire en tout temps les paramètres de nouveaux types de test, ou de reprogrammer des tests existants.

Le module 22 est lié à une interface 19, par exemple une prise USB, une interface sans-fil de type Bluetooth ou autres, etc.

L'accéléromètre 1 permet uniquement de mesurer des accélérations selon trois axes, et en particulier la valeur de l'accélération selon la direction de déplacement présumée de la charge.

La suite de données d'accélération obtenue au cours d'un test permet de déterminer facilement d'autres grandeurs, notamment :

- La force $F = m \cdot (g + a)$, g étant l'accélération due à la gravité et a l'accélération impartie par l'athlète.

- La vitesse $v = \int a \cdot dt$.

- La puissance $P = \dfrac{dE}{dt} = F \cdot v = m \cdot a \cdot v$, par exemple la puissance d'impulsion, la puissance de poussée, la puissance de freinage, etc.

- Une distance, par exemple la hauteur d'un saut, par intégration de la vitesse.

- La durée de certaines phases d'un test, par exemple un temps de contact, un temps de vol, etc.

- L'indice de réactivité.

- L'indice de rigidité.

- D'autres exemples sont donnés dans la suite de la description ou peuvent être imaginés par l'homme du métier.

Les formules ci-dessus sont valables dans le cas où l'accélération et la vitesse sont parallèles, par exemple lors de déplacements purement verticaux. En cas de vecteurs non parallèles, le calcul doit être effectué vectoriellement, ou de préférence en effectuant des produits scalaires qui ne tiennent compte que de la projection de l'accélération le long de l'axe de déplacement, par exemple de l'axe vertical.

Par ailleurs, il est aussi possible de calculer des forces, vitesses et puissances maximales ou moyennes au cours d'un test, d'un mouvement ou d'une phase de chaque mouvement. Ainsi, à partir d'une simple suite de mesure d'accélérations, il est possible de déduire toute une série de paramètres physiologiques, de les comparer dans le temps et de les comparer entre différents athlètes.

Les calculs sont de préférence effectués immédiatement après la phase d'acquisition de données d'accélération lors du test. Dans une variante, au moins une partie des calculs est effectuée au cours des tests, et/ou pendant les pauses entre deux mouvements, afin par exemple de réduire le temps de calcul à la fin du test. Dans une variante préférentielle, les calculs nécessaires afin de déterminer des conditions d'erreur ou les signaux acoustiques ou visuels émis au cours du test sont effectués sans attendre la fin, tandis que le calcul des autres valeurs physiologiques musculaires est effectué après la fin de chaque test ou de chaque mouvement dans un test comportant plusieurs mouvements.

Dans un exemple, on calcule par exemple la vitesse momentanée tout au long du test afin de réagir, par exemple avec un signal d'erreur, si l'utilisateur déplace une charge dans la mauvaise direction ou n'effectue pas le mouvement attendu. Les calculs lors du test permettent aussi de distinguer entre les différents mouvements d'un test et/ou entre les différentes phases d'un mouvement. De manière générale, l'accéléromètre peut utiliser une connaissance a priori des grandeurs mesurées ou calculées en fonction du test pour aider l'utilisateur et/ou pour générer des signaux d'erreur si les grandeurs obtenues ne correspondent pas à un motif dépendant du test (par exemple si la vitesse est positive au lieu d'être négative, si l'accéléromètre n'est pas immobile, etc.)

Il est aussi possible d'approximer la vitesse, ou d'autres valeurs, au cours du test, et de recalculer ces grandeurs plus précisément à la fin du test, ou entre deux mouvements.

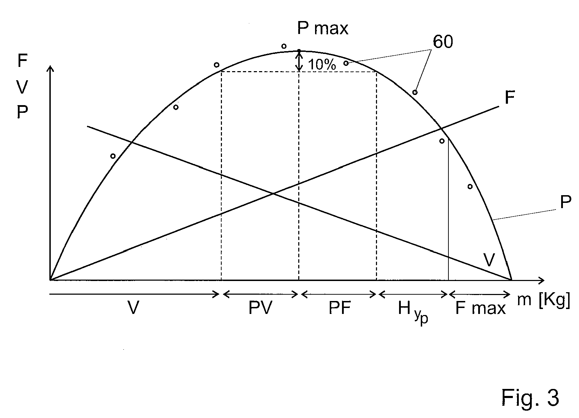

La figure 3 illustre de manière schématique une évolution possible de la force F, de la vitesse V et de la puissance P déployée par un athlète qui soulève différentes charges de masse m variable. La force gravitationnelle (m X g) exercée sur la charge déployée augmente linéairement avec la masse de la charge. En revanche, la vitesse de lever V diminue avec l'augmentation de la masse soulevée ; l'athlète soulève plus rapidement des masses légères.

La puissance P au cours de l'effort passe donc par un optimum pour une valeur donnée de la masse m soulevée. La relation P = f (m) peut être obtenue par exemple par des calculs d'interpolation ou d'extrapolation à partir d'une série de test (profil) effectués avec des charges variables, permettant d'obtenir la courbe P à partir de résultats de mesure 60 illustrés.

Cette courbe de puissance qui peut être calculée ou prise en compte par l'accéléromètre est notamment utile afin de déterminer la charge avec laquelle l'athlète doit s'entraîner pour obtenir différents types de résultats. Un gain de puissance maximale peut être obtenu en s'entraînant avec la valeur de la masse correspondant à Pmax. Un athlète souhaitant améliorer sa vitesse s'entraînera de préférence dans le domaine d'entraînement V, c'est-à-dire avec une masse légère soulevée avec moins de 10% de la puissance maximale. Le domaine PV (Puissance et Vitesse) correspond à des masses plus légères que la masse optimale et nécessitant au moins 90% de la puissance maximale. PF (Puissance Force) nécessite aussi entre 90 et 100 % de la puissance maximale, mais avec des masses plus lourdes. Une hypertrophie musculaire pour des adeptes du body-building sera obtenue en soulevant lentement des masses lourdes, la puissance développée étant alors inférieure à 90% de la puissance maximale (gamme de valeurs Hyp).

On voit ainsi que la détermination de la puissance développée pour soulever différentes masses permet de déterminer les conditions d'entraînement optimales pour un athlète.

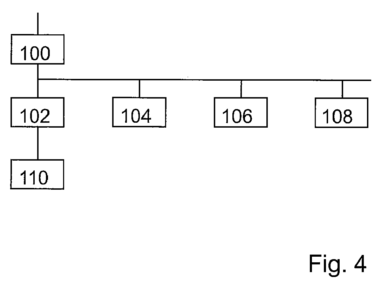

L'architecture du logiciel permet donc à l'utilisateur de choisir entre quelques tests simples, rapides à exécuter, et qui donnent des renseignements physiologiques permettant aux sportifs ou aux entraineurs d'améliorer les plans d'entraînement. Le logiciel permet de calculer et d'afficher des grandeurs obtenues à partir de tests simples, indépendants du type de sport pour lequel le sportif s'entraîne. La figure 4 est un diagramme de flux qui illustre les étapes initiales exécutées par le dispositif programmé 16 suite à la mise sous tension de l'accéléromètre. Au cours de l'étape 100, le dispositif 16 exécute une routine de démarrage qui aboutit à l'affichage du menu illustrés sur la figure 1. L'utilisateur peut sélectionner au cours des étapes suivantes 102 à 108 une des positions du menu 51 à 54 pour exécuter une action. Les positions de menu illustrées sur la figure peuvent être remplacées par des icônes correspondantes.

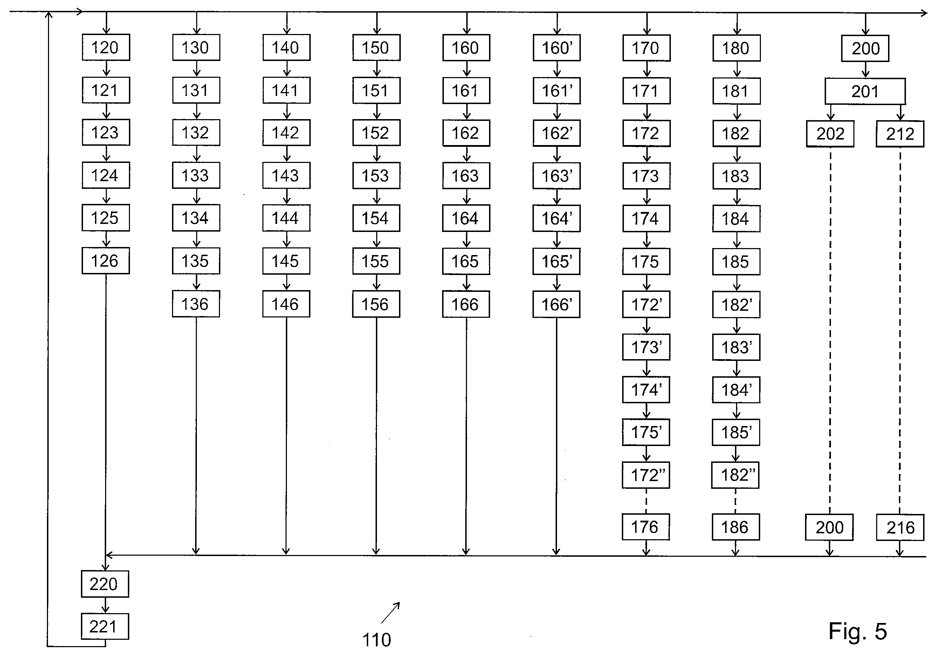

En sélectionnant au cours de l'étape 102 la position de menu 51, l'utilisateur est invité à sélectionner un test puis à effectuer ce test. La routine 110 correspondante sera décrite plus en détail en relation avec la figure 5.

Le choix de la position de menu 52 (étape 104) permet de consulter les résultats de mesure de test sauvés auparavant. L'utilisateur peut ainsi vérifier ses performances précédentes, ou celles d'autres utilisateurs enregistrés dans le système.

La position de menu 53 (choisie au cours de l'étape 106) permet d'introduire ou de modifier les paramètres généraux de l'accéléromètre, par exemple la langue des menus, la date, l'heure, le choix des unités (métriques ou pouces/livres), etc. Les modifications se font de préférence en introduisant un choix dans une liste au moyen des organes 13A, 13B.

La position de menu 54 (étape 108) permet d'introduire et de modifier des paramètres propres aux utilisateurs. Le système permet de préférence d'introduire plusieurs utilisateurs avec des paramètres associés; les utilisateurs s'identifient avant chaque utilisation et les résultats de mesure sont associés et sauvegardés en relation avec un utilisateur.

Au cours de l'étape 108, l'utilisateur peut introduire des paramètres propres à chaque utilisateur tels que :

- Nom, pseudonyme ou numéro de l'utilisateur

- Sexe

- Poids

- Taille

- Catégorie (amateur, débutant, professionnel, etc.)

- Eventuellement autres préférences (poids des barres, etc.)

Ces paramètres sont de préférence indépendants des tests et peuvent être stockés dans des positions mémoires, par exemple dans une mémoire Flash, puis utilisés pour les calculs associés à différents types de tests.

Lorsque l'utilisateur choisit la position de menu 51 pour effectuer un test, il est invité à choisir au cours de l'étape 110, dans une liste et à l'aide des organes 13A, 13B le type de test qu'il souhaite effectuer. La figure 5 illustre de manière plus détaillée le déroulement des tests que l'utilisateur est invité à effectuer après avoir choisi la position de menu 51.

L'étape 120 est entamée lorsque l'utilisateur sélectionne le test général d'entraînement ( « trainer test » ). Le capteur est lié à l'athlète, ou à la charge déplacée, d'une manière qui dépend du test effectivement réalisé. Cette position de menu est destinée notamment à la mesure de quelques paramètres physiologiques peu spécifiques à partir de tests qui ne font pas l'objet d'un menu spécifique.

Au cours de l'étape 121, un message sur l'écran 11 invite l'utilisateur à attendre que la référence de l'axe verticale donné par la gravitation ait été prise correctement. Le test démarre ensuite en appuyant sur la touche 13B (étape 123). Le test démarre après cette pression seulement si l'accéléromètre est maintenu au moins trois secondes immobile dans une position verticale, utilisée pour orienter correctement l'accéléromètre dans l'espace afin de déterminer la direction verticale. Dans le cas contraire, un signal d'erreur est émis si l'athlète ne se tient pas immobile durant au moins trois secondes.

Le début de la mesure est signalé à l'utilisateur par un signal acoustique, par exemple un bip long, qui l'invite à commencer le test prévu. L'utilisation de signaux acoustiques permet de supprimer la nécessite de regarder l'écran durant les exercices.

Dans ce test, des données d'accélération selon la direction verticale sont obtenues à une cadence de 200 ou de 500 Hertz (étape 125), la cadence pouvant éventuellement être modifiée dans les paramètres généraux ou adaptée à la mémoire disponible. La fin des mesures est signalée par un signal acoustique, par exemple un bip répétitif. Dans ce test, l'accéléromètre détermine que le test est terminé lorsque l'utilisateur appuie à nouveau sur la touche 13B ou au plus tard après une durée déterminée, par exemple 30 secondes lors d'un échantillonnage à 500 Herz ou 60 secondes lors d'un échantillonnage à 200 hertz (selon les paramètres choisis par l'utilisateur). Le test est aussi interrompu avec un signal d'erreur si l'accéléromètre détecte des accélérations improbables.

A la fin des mesures (étape 126), des valeurs calculées au cours et/ou à l'issue des mesures sont affichées sur l'écran 11. Ce test peut utiliser le poids de l'utilisateur identifié, ou la masse soulevée, pour calculer la force concentrique, la vitesse de mouvement et la puissance déployée par l'utilisateur au cours du test, et pour afficher ensuite les maximas de ces trois valeurs. Des indications quant aux progrès éventuellement obtenus depuis les derniers tests sauvés peuvent être affichées, ainsi que des notes ou des appréciations sur les performances de l'athlète (niveau bon, confirmé, etc.).

Comme pour les autres tests, le programme demande ensuite au cours de l'étape 220 si l'utilisateur souhaite sauvegarder (étape 221) les résultats de mesure. Dans une variante préférentielle, seuls les résultats calculés sont sauvegardés afin d'économiser de la place mémoire. Il est cependant aussi possible de sauvegarder la séquence complète de données d'accélération obtenues au cours du test, et/ou des résultats de calculs intermédiaires. Un stockage de toutes les valeurs d'accélération permet par exemple un transfert de ces données vers un ordinateur externe et de nouveaux calculs ou traitements plus élaborés, ou un affichage graphique de la séquence de données d'accélération ou d'autres valeurs obtenues.

Les étapes 130 à 136 correspondent au choix du test développé-couché (« bench press »). Dans ce test, l'utilisateur est couché sur le dos et soulève une ou plusieurs fois des haltères avec les bras. L'accéléromètre est fixé de préférence sur la barre des haltères.

Après la sélection du test 130, des indications sont affichées au cours de l'étape 131 pour inviter l'utilisateur à placer l'accéléromètre correctement puis à entrer la masse des haltères soulevés (étape 132). La masse est de préférence choisie au moyen des boutons 13A, 13B dans une liste à choix multiple pré-établie. Dans une variante, l'utilisateur peut incrémenter ou décrémenter la valeur de masse proposée avec les touches 13A, puis confirmer la valeur choisie avec la touche 13B.

Le test démarre ensuite au cours de l'étape 133 lorsque l'utilisateur appuie sur la touche 13B, et à condition que l'accéléromètre ait ensuite détecté un mouvement (lorsque l'utilisateur saisit la barre) suivi trois secondes d'immobilité.

Le début de l'acquisition est signalé à l'utilisateur par un message d'instructions affiché qui l'invite à saisir la barre puis à rester immobile. Un message d'erreur est émis après 5 secondes si l'accéléromètre n'est pas resté immobile pendant au moins trois secondes consécutives. Dans le cas contraire, un signal acoustique, par exemple un bip long, est émis pour inviter l'athlète à commencer le test prévu et à soulever puis reposer les haltères cinq fois de suite. Des instructions correspondantes sont affichées.

L'orientation de l'accéléromètre par rapport à la verticale est déterminée au cours des trois secondes d'immobilisme.

Un signal acoustique est de préférence émis entre chaque mouvement, lorsque l'utilisateur a reposé la barre pendant une seconde, pour l'inviter à effectuer le mouvement suivant.

Dans ce test, des données d'accélération selon la direction verticale sont obtenues de préférence à une cadence de 200 Hertz (étape 135). La fin des mesures est signalée après la fin du dernier mouvement par un signal acoustique, par exemple un bip répétitif. Le test est interrompu avant la fin du test avec un signal d'erreur par exemple si l'utilisateur laisse les haltères immobiles durant plus de cinq secondes entre deux mouvements, ou si les déplacements ne sont pas dirigés dans la direction attendue (par exemple parce que le test n'est pas démarré en position basse), ou si le nombre de cycles est inférieur à 5, ou encore lorsque le déplacement dure plus de cinq secondes.

A la fin des mesures (étape 136), des valeurs calculées au cours et/ou à l'issue des mesures sont affichées sur l'écran 11. Ce test utilise le poids des haltères pour calculer et afficher la puissance impulsionnelle, la vitesse de mouvement moyenne et la force concentrique moyenne déployée par l'utilisateur au cours du test. Dans un mode de réalisation préférentiel, les moyennes sont calculées sur les trois meilleurs mouvements dans la série de cinq, c'est-à-dire les trois mouvements au cours desquels la puissance impulsionnelle maximale a été déployée. Des tendances peuvent être affichées, par exemple avec des flèches, pour indiquer les progrès par rapport à des résultats de tests précédents enregistrés. L'utilisateur peut stocker ces résultats.

Les étapes 140 à 146 correspondent au choix du test demi squat qui permet de tester des groupes musculaires dans les jambes et le bassin. L'athlète doit plier cinq fois les jambes puis se relever en gardant le dos droit ; l'accéléromètre est fixé contre le tronc ou sur une barre d'haltères soulevée par l'athlète. Le déroulement du test est similaire à celui du développé-couché décrit en relation avec les éléments 130 à 136 de la figure 5. Des résultats similaires sont calculés puis affichés au cours de l'étape 146, en prenant en compte le poids de l'athlète et la charge éventuelle portée par l'athlète.

Les étapes 150 à 156, 160 à 166 et 160' à 166' illustrent trois tests de saut. Les étapes 150 à 156 correspondent au choix d'un test de pliométrie (réactivité) afin de mesurer les qualités de détente et d'élasticité de l'athlète. L'utilisateur doit par exemple effectuer un saut vertical sans flexion des genoux et sans élan.

Les étapes 160 à 166 correspondent en revanche au choix d'un saut vertical avec élan « countermovement jump » : l'utilisateur est autorisé à effectuer une flexion des genoux avant l'extension qui le propulse verticalement.

Les étapes 160' à 166' servent en revanche à mesurer la détente sèche, non pliométrique, d'un utilisateur qui commence le test avec le genou fléchi à 90° puis effectue une poussée maximale vers le haut. Les mains sont sur les hanches pour éviter une participation des bras. Ce test est appelé « squatt jump ».

D'autres types de tests de sauts peuvent être prévus.

Dans tous les tests de saut, la fréquence d'acquisition est de préférence élevée, par exemple de 500 Hertz. Le type de saut choisi est affiché au cours de l'étape 151, 161 resp. 161'. Au cours de l'étape 152 (resp. 162; 162'), le poids de l'utilisateur mémorisé préalablement est affiché ; l'utilisateur a la possibilité de l'incrémenter ou de le décrémenter avec les touches 13A, 13B. L'utilisateur est ensuite invité à presser sur une touche pour démarrer le test (étape 153/163/163'), qui démarre avec un signal acoustique dès qu'une durée d'immobilisation de trois secondes a été détectée (154/164/164').

L'utilisateur est ensuite invité à effectuer cinq sauts verticaux dans les conditions correspondantes à chaque test (155/165/165'). Le test est interrompu avec un message d'erreur si le nombre de sauts est inférieur à cinq, ou si l'accéléromètre reste plusieurs secondes immobile entre deux sauts, ou si la durée des sauts est trop importante. Par ailleurs, dans le test de counterjump, un message d'erreur est aussi émis si aucun mouvement vertical vers le bas n'est détecté lorsque l'utilisateur devrait prendre son élan au début de chaque mouvement. Au contraire, dans le test de squatt jump, un message d'erreur est émis si un mouvement vertical vers le bas est détecté avant le saut. Dans le test de pliométrie, une autre erreur est générée si le temps de contact au sol est supérieur à 0,25 secondes. Par ailleurs, un signal acoustique peut être émis entre chaque mouvement.

Les résultats du test sont calculés et affichés au cours des étapes 156 respectivement 166 ou 166'. L'accéléromètre calcule et affiche éventuellement les paramètres suivants :

- Nombre de mouvements (de sauts) effectués ; comme mentionné, un signal d'erreur est émis si l'athlète n'effectue pas le nombre de sauts prévus durant le temps imparti prédéterminé.

Pour le test de pliométrie, l'accéléromètre calcule et de préférence affiche en outre les grandeurs suivantes :

- Temps de vol de chaque saut / moyenne des temps de vol / temps de vol maximal

- Temps de contact au sol entre chaque saut

- Hauteur atteinte, moyenne des hauteurs sur les 5 sauts.

- Moyenne sur trois ou cinq mouvements de l'indice de rigidité musculaire (stiffness) S défini comme

$$S = (\text{force à vitesse nulle}) \text{ divisé par la distance d'abaissement.}$$

- Moyenne sur trois ou cinq mouvements de l'indice de réactivité R défini comme

$$R = (\text{temps de vol})^2 / \text{temps de contact au sol}$$

Dans le cas d'un saut vertical avec élan, on calculera et de préférence affichera les valeurs suivantes :

- La vitesse et la hauteur de vol maximales, et la moyenne de ces valeurs sur tous ou sur les trois meilleurs sauts.

- La puissance de freinage BP pour chaque saut, et la moyenne de la puissance de freinage sur tous ou sur les trois meilleurs sauts.

- La puissance de poussée vers le haut PP pour chaque saut, et la moyenne de la puissance de poussée sur tous ou sur les trois meilleurs sauts.

- Le rapport de puissance PR = PP / BP

D'autres valeurs peuvent être affichées pour chaque type de saut, y compris la vitesse moyenne ou maximale, des tendances, etc. Les moyennes et les maximaux peuvent être calculés sur le ou les meilleurs sauts, sur tous les sauts ou sur tous les sauts sauf le premier et/ou le dernier.

Dans le cas d'un saut vertical sans élan (squatt jump), on calculera et de préférence affichera les valeurs suivantes :

- La vitesse et la hauteur de vol maximales, et la moyenne de ces valeurs sur tous ou sur les trois meilleurs sauts.

- La puissance impulsionnelle IP pour chaque saut, et la moyenne de la puissance impulsionnelle sur tous ou sur les trois meilleurs sauts.

- La puissance de poussée vers le haut PP pour chaque saut, et la moyenne de la puissance de poussée sur tous ou sur les trois meilleurs sauts.

- Le rapport de puissance PR = PP / BP

D'autres valeurs peuvent être affichées pour chaque type de saut, y compris la vitesse moyenne ou maximale, des tendances, etc. Les moyennes et les maximaux peuvent être calculés sur le ou les meilleurs sauts, sur tous les sauts ou sur tous les sauts sauf le premier et/ou le dernier.

Les étapes 170 à 176 illustrent un test plus complexe effectué pour obtenir un profil de développé couché afin de mesurer des valeurs physiologiques sur des groupes musculaires liés aux bras. Dans ce test, l'utilisateur effectue plusieurs (par exemple huit) mouvements de charge comme dans le test 130-136, mais en variant la charge entre chaque mouvement. La charge initiale et la variation de charge entre chaque mouvement dépend du niveau de l'utilisateur (déterminé par exemple à l'aide de tests précédents), de son sexe et éventuellement de son poids ou d'autres paramètres.

Les étapes 170 à 174 correspondent respectivement aux étapes 120 à 124 du test de développé couché ordinaire, seules les indications affichées au cours de l'étape 171 étant différentes. Au cours de l'étape 175, l'utilisateur doit soulever une seule fois la charge, au lieu de cinq fois dans le test 125. Il est aussi possible de soulever la charge plusieurs fois dans le test 175.

A l'issue du mouvement 175, au lieu d'afficher immédiatement les résultats, l'utilisateur est invité à modifier la charge (172'), à confirmer le remplacement avec la touche 13B (173') puis à recommencer l'exercice (175') après le signal acoustique 174'. Ce processus complet est répété plusieurs fois, par exemple huit fois, avec des charges à chaque fois variables. Les résultats du test complet sont ensuite calculés puis affichés au cours de l'étape 176.

Les résultats calculés et/ou affichés comprennent par exemple :

- Le nombre de mouvements effectués

- La puissance maximale PMax, obtenue par exemple à l'aide d'une interpolation par approximation polynomiale des valeurs de puissance moyenne ou maximale de chaque mouvement

- La charge optimale avec laquelle l'athlète devrait s'entraîner pour développer la puissance PMax, selon la courbe illustrée sur la figure 3.

- La charge maximale que l'athlète parviendrait à soulever à une vitesse minimale donnée, par exemple 10 cm/s.

- La résistance maximale

- Les limites des domaines V, PV, PF, Hyp sur la figure 3

- Etc.

Les étapes 180 à 186 correspondent au choix du test « profil demi squat » afin de mesurer des valeurs physiologiques sur des groupes musculaires liés aux jambes. Le déroulement du test est similaire à celui décrit en relation avec les étapes 170 à 176, mais l'utilisateur est invité à effectuer plusieurs mouvements de demi squats en poussant des charges variables, au lieu d'une série de développé couchés. Les résultats calculés puis affichés sont par exemple les mêmes que pour le profil développé couché.

Les étapes 200 à 216 correspondent à l'exécution de tests définis par l'utilisateur et par exemple chargés via une interface depuis un ordinateur externe. De nouveaux tests peuvent être programmés par l'utilisateur à l'aide d'un programme adapté sur son PC, et/ou téléchargés depuis Internet. La définition des tests est par exemple déterminée par un fichier XML ou par une autre structure de données adaptée pour définir les messages visuels et sonores émis, les valeurs calculées et affichées, etc. Dans une variante, des tests peuvent aussi être crées ou modifiés depuis l'accéléromètre 1.

Après avoir effectué le choix 200, l'utilisateur est invité à choisir quel test programmé il souhaite effectuer parmi une liste de tests installés précédemment. Dans l'exemple, il a le choix entre deux tests supplémentaires commençant par les étapes 202 et 212. La suite des étapes dépend de la programmation, mais se termine typiquement par un affichage de résultats au cours des étapes 206 respectivement 216, les grandeurs calculées et affichées étant aussi déterminées par le fichier téléchargé depuis l'ordinateur externe.

Dans les exemples ci-dessus, la fin du test et le passage à l'étape de calcul et d'affichage des résultats est déterminé automatiquement après l'exécution d'un nombre de mouvements (par exemple un lever de charge ou un saut) dépendant du test. L'exécution complète d'un mouvement peut être déterminée à partir des données obtenues par l'accéléromètre (pas exemple en détectant que l'accéléromètre est immobile) ou après l'écoulement d'une durée prédéterminée qui peut aussi dépendre du test. Il est aussi possible de prévoir des tests de durée prédéterminée, par exemple des tests consistant à effectuer le maximum de sauts, de levers ou de gainages dans un temps donné.

La plupart des tests décrits à titre d'exemple ci-dessus comportent plusieurs mouvements précédés et séparés par des intervalles au cours desquels l'accéléromètre est immobile. Un signal d'erreur peut être émis si l'accéléromètre n'est pas immobile. Il est aussi possible d'utiliser ces phases pour calibrer la position verticale de l'accéléromètre afin de calculer des accélérations et d'autres grandeurs uniquement dans la direction verticale, ou dans la direction de déplacement de la charge. Dans une variante préférentielle, ces phases d'immobilisation sont aussi utilisées pour réinitialiser la vitesse et/ou la position de l'accéléromètre afin d'éviter le cumul des erreurs dues à l'intégration.

La sélection initiale du type de test que souhaite effectuer l'utilisateur détermine par exemple les paramètres suivants :

- Fréquence d'acquisition des données d'accélération, ainsi que éventuellement le nombre de bits employé.

- Indications initiales affichées à l'utilisateur au début du test, et/ou lors du test, et/ou entre différents mouvements.

- Paramètres devant être introduits par l'utilisateur, et/ou lus depuis une position mémoire.

- Signaux sonores restitués au début du test, et/ou lors du test, et/ou entre différents mouvements.

- Conditions pour démarrer l'acquisition.

- Conditions qui vont générer une erreur et/ou interrompre l'acquisition durant un test.

- Conditions qui vont déterminer la fin d'un test, et/ou la fin d'un mouvement dans un test comportant plusieurs mouvements.

- Profil de grandeurs mesurées ou calculées lors du test, ce profil pouvant par exemple être utilisé pour générer des erreurs si les données acquises ne correspondent pas aux attentes.

- Valeurs calculées au cours des tests, et/ou entre deux mouvements d'un test, et/ou à la fin d'un test.

- Valeurs affichées pendant et/ou au terme d'un test.

- Valeurs sauvegardées en mémoire.

- Etc.

Aucun de ces paramètres n'est essentiel. Il est donc possible de mettre en oeuvre des procédés dans lequel n'importe quelle sous-combinaison de paramètres dépend du test sélectionné.

**Numéros de référence employés sur les figures**

| 1 | Accéléromètre |
|---|---|
| 10 | Boîtier |
| 11 | Affichage |
| 13A | Organes de commande : boutons de navigation |
| 13B | Organes de commande : bouton de confirmation |
| 14 | Capteur d'accélération |
| 15 | Batterie ou accumulateur |
| 16 | Dispositif programmé, par exemple microprocesseur |
| 17 | Transducteur sonore |
| 19 | Prise USB |
| 20 | Horloge |
| 21 | Convertisseur analogique-numérique |
| 22 | Module d'entrée sortie |
| 23 | Mémoire |
| 51 | Position de menu pour la sélection de choix de test |
| 52 | Position de menu pour la visualisation de résultats |
| 53 | Position de menu pour la sélection de paramètres généraux |
| 54 | Position de menu pour la sélection de paramètres d'utilisateurs |
| 55 | Affichage état de la batterie |
| 56 | Affichage état de la mémoire |
| 60 | Résultats de mesure de test de profil |

**Revendications**

1. Procédé pour obtenir les valeurs physiologiques musculaires d'un utilisateur à l'aide d'un accéléromètre programmable, le procédé comportant les opérations suivantes :

   - sélection (110) par l'utilisateur du type de test à effectuer;
   - mesures d'une séquence de données d'accélération au cours du test(125, 135, 145, 155, 165, 175, 185, ..)
   **caracterisé en ce que** le procédé comporte en outre les étapes suivantes:
   - émission d'un signal sonore à la fin du test, la fin du test étant déterminée en vérifiant une condition dépendant du type de test sélectionné ;
   - affichage (126, 136, 146, 156, 166, 176, 186) d'une valeur calculée à partir desdites mesures successives d'accélération et dépendant du type de test sélectionné.

2. Le procédé de la revendication 1, dans lequel la fin du test dépend pour certains tests de l'écoulement d'un temps déterminé,
   dans lequel pour d'autres tests la fin dépend de l'exécution d'un nombre de mouvements déterminé,
   et dans lequel pour encore d'autres tests la fin dépend de l'exécution d'un nombre de mouvements déterminé avec des charges variables.

3. Le procédé de l'une des revendications 1 à 2, dans lequel pour au moins certains tests la fin du test dépend de l'écoulement d'une durée qui dépend du test sélectionné.

4. Le procédé de l'une des revendications 1 à 3, dans lequel au moins un test implique l'exécution de plusieurs mouvements, la fin du test dépendant du nombre de mouvements effectués.

5. Le procédé de l'une des revendications 1 à 4, dans lequel pour au moins certains tests la fin du test dépend d'un nombre de mouvements effectués, un signal étant émis entre chaque mouvement pour inviter l'utilisateur à effectuer chaque mouvement.

6. Le procédé de l'une des revendications 1 à 5, dans lequel la fréquence d'acquisition de l'accélération dépend du type de test sélectionné.

7. Le procédé de l'une des revendications 1 à 6, dans lequel le nombre de valeurs d'accélération acquises dépend du type de test sélectionné.

8. Le procédé de l'une des revendications 1 à 7, dans lequel un signal d'erreur est émis lorsqu'une grandeur obtenue à partir de la séquence de valeurs d'accélération mesurée ne correspond pas à un motif dépendant du type de test sélectionné.

9. Le procédé de l'une des revendications 1 à 8, dans lequel au moins un test comprend plusieurs mouvements séparés les unes des autres par des signaux dépendant du type de test sélectionné.

10. Le procédé de l'une des revendications 1 à 9, dans lequel un calcul dépendant du type de test sélectionné est effectué à partir desdites données d'accélération.

11. Le procédé de l'une des revendications 1 à 10, dans lequel une valeur dépendant du type de test sélectionné est affichée au cours ou au terme du test.

12. Le procédé de l'une des revendications 1 à 11, comprenant une étape au cours de laquelle au moins un paramètre dépendant du type de test sélectionné est demandé à l'utilisateur ou lu depuis une position mémoire.

13. Le procédé de l'une des revendications 1 à 12, dans laquelle une séquence de données de vitesse est déterminée au cours du test par intégration de ladite séquence de données d'accélération,
    la vitesse étant réinitialisée au moins une fois lors du test lorsque l'accéléromètre détermine que la vitesse est nulle.

14. Le procédé de la revendication 13, ledit test comportant plusieurs mouvements séparés par des phases d'immobilisation, la vitesse étant réinitialisée au cours d'au moins une dite phase d'immobilisation.

**15.** Le procédé de l'une des revendications 1 à 14, comportant une étape de détermination de la force maximale ou de la force moyenne à partir desdites données d'accélération multipliées par un paramètre de masse introduit par l'utilisateur.

**16.** Le procédé de la revendication 15, comportant une étape de détermination de la puissance maximale ou de la puissance moyenne à partir de la force et de la vitesse.

**17.** Le procédé de l'une des revendications 1 à 16, comportant une étape de détermination d'une hauteur de saut et/ou d'une durée de vol à partir de ladite séquence de données d'accélération.

**18.** Le procédé de l'une des revendications 1 à 17, comportant une étape de détermination de l'explosivité musculaire à partir de ladite séquence de données d'accélération.

**19.** Le procédé de l'une des revendications 1 à 18, comportant l'émission de signaux sonores au début du test, respectivement en cours de test, afin de signaler un début de test, respectivement une action à effectuer ou une erreur en cours de test.

**20.** Accéléromètre portable pour la mesure de valeurs physiologiques triaxiales, comportant :

> un capteur d'accélération (14) ;
> un affichage (11) ;
> une batterie électrique (15) ;
> des organes (13, 13A, 13B) permettant l'introduction manuelle de paramètres et/ou de commandes ;
> un dispositif programmé (16) pour inviter l'utilisateur à sélectionner au moyen desdits organes un type de test parmi plusieurs tests prédéfinis, pour émettre un signal à la fin du test déterminée en fonction du type de test sélectionné, et pour effectuer des calculs à partir d'une séquence de données d'accélération obtenues pendant le test et dépendant du type de test sélectionné.

**21.** L'accéléromètre de la revendication 20, ledit capteur d'accélération (14) permettant de mesurer l'accélération selon plusieurs axes, seule l'accélération selon une direction privilégiée déterminée lorsque l'accéléromètre est immobile étant utilisée pour lesdits calculs.

**22.** L'accéléromètre de l'une des revendications 20 à 21, comportant une interface (19,22) pour le connecter à un ordinateur externe afin de charger de nouveaux tests et/ou de transférer dans l'ordinateur externe les résultats du test.

**Patentansprüche**

**1.** Verfahren zur Erhaltung von physiologischen Muskelwerten eines Benutzers mittels eines programmierbaren Beschleunigungsmessers, worin das Verfahren die folgenden Operationen umfasst:

> - Auswahl (110), durch den Benutzer, des Typs des auszuführenden Tests;
> - Messungen einer Sequenz von Beschleunigungswerten während des Tests (125, 135, 145, 155, 165, 175, 185, ...);

> **dadurch gekennzeichnet, dass** das Verfahren zudem die folgenden Schritte umfasst:

> - Abgabe eines Tonsignals am Ende des Tests, wobei das Ende des Tests durch die Verifizierung einer Bedingung abhängig vom Typ des ausgewählten Tests bestimmt wird;
> - Anzeigen (126, 136, 146, 156, 166, 176, 186) eines Werts, berechnet auf Grundlage der besagten sukzessiven Messungen der Beschleunigung und abhängig vom Typ des ausgewählten Tests.

**2.** Das Verfahren von Anspruch 1, worin das Ende des Tests für bestimmte Tests vom Verstreichen eines vorgegebenen Zeitraums abhängt,
worin für andere Tests das Ende des Tests von der Ausführung einer Anzahl von vorgegebenen Bewegungen abhängt,
und worin für weitere Tests das Ende von der Ausführung einer Anzahl von vorgegebenen Bewegungen mit variablen Belastungen abhängt.

3. Das Verfahren eines der Ansprüche 1 bis 2, worin mindestens für bestimmte Tests das Ende des Tests vom Verstreichen eines vom ausgewählten Test abhängigen Zeitraums abhängt.

4. Das Verfahren eines der Ansprüche 1 bis 3, worin mindestens ein Test die Ausführung von mehreren Bewegungen umfasst, wobei das Ende des Tests von der Anzahl von ausgeführten Bewegungen abhängt.

5. Das Verfahren eines der Ansprüche 1 bis 4, worin mindestens für bestimmte Tests das Ende des Tests von der Anzahl von ausgeführten Bewegungen abhängt, wobei zwischen jeder Bewegung ein Signal abgegeben wird, um den Benutzer zur Ausführung jeder Bewegung aufzufordern.

6. Das Verfahren eines der Ansprüche 1 bis 5, worin die Frequenz des Erfassens der Beschleunigung vom Typ des ausgewählten Tests abhängt.

7. Das Verfahren eines der Ansprüche 1 bis 6, worin die Anzahl der erfassten Beschleunigungswerte vom Typ des ausgewählten Tests abhängt.

8. Das Verfahren eines der Ansprüche 1 bis 7, worin ein Fehlersignal abgegeben wird, wenn ein aus der Sequenz der gemessenen Beschleunigungswerte erhaltener Wert nicht einem vom Typ des ausgewählten Tests abhängigen Muster entspricht.

9. Das Verfahren eines der Ansprüche 1 bis 8, worin mindestens ein Test mehrere durch von dem Typ des ausgewählten Tests abhängige Signale voneinander getrennte Bewegungen umfasst.

10. Das Verfahren eines der Ansprüche 1 bis 9, worin eine vom Typ des ausgewählten Tests abhängige Berechnung aufgrund der besagten Beschleunigungsdaten durchgeführt wird.

11. Das Verfahren eines der Ansprüche 1 bis 10, worin ein vom Typ des ausgewählten Tests abhängiger Wert während des Tests oder an dessen Ende angezeigt wird.

12. Das Verfahren eines der Ansprüche 1 bis 11, mit einem Schritt während dessen mindestens ein vom Typ des ausgewählten Tests abhängiger Parameter vom Benutzer verlangt oder von einer Speicherposition gelesen wird.

13. Das Verfahren eines der Ansprüche 1 bis 12, worin eine Sequenz von Geschwindigkeitsdaten während des Tests durch die Integration der besagten Sequenz von Beschleunigungsdaten bestimmt wird,
wobei die Geschwindigkeit während des Tests mindestens einmal neu initialisiert wird, wenn der Beschleunigungsmesser bestimmt, dass die Geschwindigkeit null ist.

14. Das Verfahren von Anspruch 13, wobei der besagte Test mehrere durch Phasen des Stillstands unterbrochene Bewegungen aufweist, wobei die Geschwindigkeit während mindestens einer der besagten Phasen des Stillstands neu initialisiert wird.

15. Das Verfahren eines der Ansprüche 1 bis 14, mit einem Schritt der Bestimmung der Maximal- oder Durchschnittskraft anhand der besagten Beschleunigungsdaten, mit einem durch den Benutzer eingegebenen Parameter für Masse multipliziert.

16. Das Verfahren von Anspruch 15, mit einem Schritt der Bestimmung der Maximal- oder Durchschnittsleistung anhand der Kraft und der Geschwindigkeit.

17. Das Verfahren eines der Ansprüche 1 bis 16, mit einem Schritt der Bestimmung der Höhe eines Sprungs und/oder der Flugdauer anhand der besagten Sequenz von Beschleunigungsdaten.

18. Das Verfahren eines der Ansprüche 1 bis 17, mit einem Schritt der Bestimmung der Muskelschnellkraft anhand der besagten Sequenz von Beschleunigungsdaten.

19. Das Verfahren eines der Ansprüche 1 bis 18, mit der Abgabe von Tonsignalen am Anfang bzw. während des Tests, um ein Beginnen eines Tests bzw. eine auszuführende Handlung oder einen Fehler während des Tests anzuzeigen.

20. Tragbarer Beschleunigungsmesser zur Messung von dreiaxialen physiologischen Werten, umfassend:

einen Beschleunigungssensor (14);
eine Anzeige (11);
eine elektrische Batterie (15);
Elemente (13, 13A, 13B) die es ermöglichen, Parameter oder Befehle manuell einzugeben;
ein programmiertes Gerät (16), das den Benutzer auffordert, mittels der besagten Elemente einen Typ eines Tests aus mehreren vorgegebenen Tests auszuwählen, das ein gemäss dem ausgewählten Test bestimmtes Signal am Ende des Tests abgibt und das Berechnungen aufgrund einer während des Tests erhaltenen Sequenz von Beschleunigungsdaten durchführt, und wobei diese vom Typ des ausgewählten Tests abhängen.

21. Der Beschleunigungsmesser von Anspruch 20, wobei der besagte Beschleunigungssensor (14) es ermöglicht, die Beschleunigung entlang mehrerer Achsen zu messen, worin nur die Beschleunigung in eine bevorzugte während des Stillstands des Beschleunigungsmessers bestimmte Richtung für besagte Berechnungen benutzt wird.

22. Der Beschleunigungsmesser eines der Ansprüche 20 bis 21, mit einer Schnittstelle (19, 22) zur Verbindung an einen externen Computer, um neue Tests zu laden und/oder die Resultate des Tests an den externen Computer zu übermitteln.

## Claims

1. Method for obtaining physiological muscle values of a user by means of a programmable accelerometer, wherein the method comprises the following operations:

   - selection (110), by the user, of the type of test to be carried out;
   - measurements of a sequence of acceleration data during the test (125, 135, 145, 155, 165, 175, 185, ...);

   **characterized in that** the method additionally comprises the following steps:

   - emission of a sound signal at the end of the test, the end of the test being determined by verifying a condition dependent on the type of test selected;
   - displaying (126, 136, 146, 156, 166, 176, 186) a value calculated on the basis of said successive measurements of the acceleration and dependent on the type of test selected.

2. The method of claim 1, wherein the end of the test depends for certain tests on the lapsing of a determined period of time,
   wherein for other tests the end depends on the execution of a determined number of movements,
   and wherein for further other tests the end depends on the execution of a determined number of movement with variable loads.

3. The method of one of the claims 1 to 2, wherein for at least certain tests the end of the test depends on the lapsing of a period of time dependent on the selected test.

4. The method of one of the claims 1 to 3, wherein at least one test involves the execution of several movements, with the end of the test depending on the number of movements performed.

5. The method of one of the claims 1 to 4, wherein for at least certain tests the end of the test depends on a number of executed movements, with a signal being emitted between each movement to invite the user to execute each movement.

6. The method of one of the claims 1 to 5, wherein the frequency of acquisition of the acceleration depends on the type of test selected.

7. The method of one of the claims 1 to 6, wherein the number of acceleration values acquired depends on the type of test selected.

8. The method of one of the claims 1 to 7, wherein an error signal is emitted when a quantity obtained from the sequence of measured acceleration values does not correspond to a pattern dependent on the type of test selected.

9.  The method of one of the claims 1 to 8, wherein at least one test comprises several movements separated from one another by signals depending on the type of test selected.

10. The method of one of the claims 1 to 9, wherein a calculation dependent on the type of test selected is performed from said acceleration data.

11. The method of one of the claims 1 to 10, wherein a value dependent on the type of test selected is displayed during or at the end of the test.

12. The method of one of the claims 1 to 11, including a step during which at least one parameter dependent on the type of test selected is required from the user or read from a memory position.

13. The method of one of the claims 1 to 12, wherein a sequence of speed data is determined during the test by integration of said sequence of acceleration data,
    the speed being re-initialized at least once during the test when the accelerometer determines that the speed is zero.

14. The method of claim 13, said test including several movements separated by phases of standstill, the speed being re-initialized during said at least one standstill phase.

15. The method of one of the claims 1 to 14, including a step of determining the maximum force or the average force from said acceleration data multiplied by a mass parameter entered by the user.

16. The method of claim 15, including a step of determining the maximum power or the average power from the strength and the speed.

17. The method of one of the claims 1 to 16, including a step of determining a height of a jump and/or a flight duration from said sequence of acceleration data.

18. The method of one of the claims 1 to 17, including a step of determining the muscle explosiveness from said sequence of acceleration data.

19. The method of one of the claims 1 to 18, including the emission of sound signals at the beginning of the test resp. during the test in order to signal the beginning of a test resp. an action to be executed or an error during the test.

20. Portable accelerometer for measuring tri-axial physiological values, comprising:

    an acceleration sensor (14);
    a display (11);
    an electric battery (15);
    elements (13, 13A, 13B) enabling parameters and/or commands to be entered manually;
    a programmed device (16) for inviting the user to select, by means of said elements, a type of test from among several predefined tests, for emitting a signal at the end of the test determined according to the type of test selected, and for performing calculations from a sequence of acceleration data obtained during the test and depending on the type of test selected.

21. The accelerometer of claim 20, said acceleration sensor (14) enabling the acceleration to be measured along several axes, wherein only the acceleration in a privileged direction determined when the accelerometer is immobile being used for said calculations.

22. The accelerometer of one of the claims 20 to 21, comprising an interface (19, 22) for connecting it to an external computer in order to load new tests and/or transfer into the external computer the results of the test.

Welcome

51      Tests
52      Memory
         Setting
53      Users
54

11

13A

10
10A

13B

13A

1

Fig. 1

Fig. 2

Fig. 3

Fig. 4

EP 2 027 817 B1

Fig. 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5788655 A
- WO 2005074795 A
- WO 03032826 A
- US 5474083 A
- US 6148280 A
- DE 446302
- US 20060191335 A
- EP 1834583 A